# EUROPÄISCHE PATENTSCHRIFT

(11) **EP 4 347 547 B1**
(45) Veröffentlichungstag und Bekanntmachung des Hinweises auf die Patenterteilung: **25.09.2024**
(21) Anmeldenummer: 22730239.5
(22) Anmeldetag: 24.05.2022
(51) Int. Cl.: C07C 67/347, C07C 69/675

(54) **HERSTELLUNG EINES ZWISCHENPRODUKTES DER SYNTHESE VON 2- CYCLODODECYL-1-PROPANOL**
PREPARATION OF AN INTERMEDIATE FOR THE SYNTHESIS OF 2- CYCLODODECYL-1-PROPANOL
PRODUCTION D'UN PRODUIT INTERMÉDIAIRE DE LA SYNTHÈSE DU 2-CYCLODODÉCYL-1-PROPANOL

(30) Priorität: 03.06.2021 EP 21177644
(43) Veröffentlichungstag der Anmeldung: 10.04.2024
(73) Patentinhaber: Miltitz Aromatics GmbH, 06803 Bitterfeld-Wolfen (DE)
(72) Erfinder: MÜLLER, Stefan, 04105 Leipzig (DE); PETRI, Andreas, 04155 Leipzig (DE); JIMENEZ-PINTO, Jhonny, 06124 Halle (Saale) (DE)
(74) Vertreter: Weidner Stern Jeschke
(86) Internationale Anmeldenummer: PCT/EP2022/063988
(87) Internationale Veröffentlichungsnummer: WO 2022/253626

(56) Entgegenhaltungen:
- DE-A1- 3 703 585
- ZHAO D ET AL: "Catalytic enantioselective intermolecular reductive aldol reaction to ketones", TETRAHEDRON LETTERS, ELSEVIER, AMSTERDAM , NL, vol. 47, no. 9, 27 February 2006 (2006-02-27), pages 1403 - 1407, XP025003616, ISSN: 0040-4039, [retrieved on 20060227], DOI: 10.1016/J.TETLET.2005.12.097
- FUJITA TSUTOMU ET AL: "A convenient preparative method of 3-hydroxy acids, and the reaction of 3-hydroxy acids with acidic materials", JOURNAL OF APPLIED CHEMISTRY AND BIOTECHNOLOGY, vol. 27, no. 5, 1 January 1977 (1977-01-01), pages 593 - 598, XP055862140, ISSN: 0375-9210, DOI: 10.1002/jctb.5020270504

## Beschreibung

### Gebiet der Erfindung

Die Erfindung betrifft die Herstellung eines tertiären Alkohols der Formel(I) 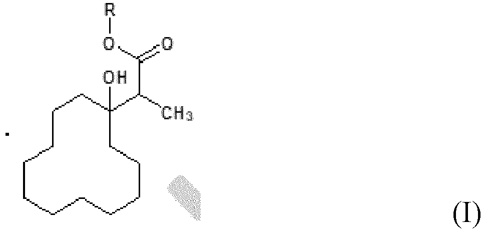 und die Herstellung von 2-Cyclododecyl-1-propanol ausgehend von diesem so hergestellten Zwischenprodukt.

### Hintergrund der Erfindung

Der kommerziell wichtige Riechstoff 2-Cyclododecyl-1-propanol (auch bekannt unter dem Namen Hydroxyambran) wird üblicherweise über zwei bis drei Reaktionsstufen ausgehend von Cyclododecanon hergestellt, wobei in der ersten Stufe das Cyclododecanon mit einem Alkyl-2-brompropionat, vorzugsweise Ethyl-2-brompropionat, in Gegenwart von Zink (Reformatsky-Reaktion) zu einem tertiären Alkohol umgesetzt wird. Durch Abspaltung von Wasser entsteht hieraus dann der entsprechende ungesättigte Ester (ein Isomerengemisch), der anschließend zum 2-Cyclododecyl-1-propanol reduziert wird.

Dieses Verfahren ist aus der Patentanmeldung DE 37 03 585 A1 bekannt.

Obgleich das herkömmliche Verfahren das Endprodukt 2-Cyclododecyl-1-propanol in guten Ausbeuten liefert, ist die erste Reaktionsstufe, also die Reformatsky-Reaktion, nicht nur mit relativ hohen Materialkosten verbunden, sondern erfordert auch aufwändige Aufarbeitungs- oder Entsorgungsarbeiten bzw. -kosten, da bei der Reaktion Zinkbromid anfällt.

Aufgabe der vorliegenden Erfindung ist es daher, ein verbessertes Verfahren zur Herstellung des Zwischenproduktes der Synthese von 2-Cyclododecyl-1-propanol, nämlich des tertiären Alkohols aus der Reformatsky-Reaktion bereitzustellen, das die beschriebenen Nachteile überwindet.

### Zusammenfassung der Erfindung

Die der Erfindung zugrundeliegende Aufgabe wird gelöst mit einem Verfahren zur Herstellung eines tertiären Alkohols mit der Formel (I) 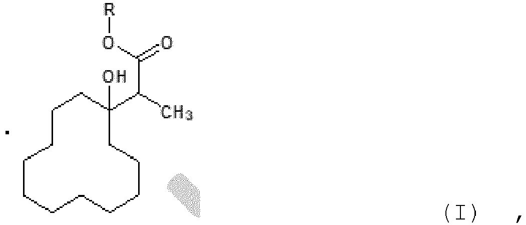 bei dem Cyclododecanon mit einem Acrylat mit der Formel RO-CO-CH=CH₂ in einer reduktiven Aldol-Reaktion unter Metall-Katalyse umgesetzt wird, wobei R in der obigen Formel (I für eine geradkettige oder verzweigte C₁-C₈-Alkyl- oder - Alkenylgruppe oder eine unsubstituierte oder substituierte Phenylgruppe steht.

Vorzugsweise wird die Reaktion unter Kupfer-Katalyse in Gegenwart eines Silans durchgeführt.

In einer bevorzugten Ausführungsform wird als Silan Polymethylhydrosiloxan (PMHS) verwendet. Es kommen aber auch andere Silane in Betracht, wie beispielsweise Tetramethylhydrosiloxan, Triethoxysilan oder Phenylsilan.

Besonders bevorzugt wird als Katalysator Cu(OAc)₂ × H₂O verwendet.

In einer Ausführungsform der Erfindung wird eine Phosphinverbindung als Ligand des Katalysators verwendet.

Dabei wird als Ligand bevorzugt Triphenylphosphin verwendet.

In einer bevorzugten Ausführungsform der Erfindung wird ein Stabilisator für das Acrylat verwendet.

Dabei wird als Stabilisator für das Acrylat bevorzugt Hydrochinonmonomethylether verwendet.

In einer weiteren bevorzugten Ausführungsform der Erfindung wird die Reaktion in einem Lösungsmittelsystem durchgeführt.

Dabei umfasst das Lösungsmittelsystem bevorzugt Toluol oder Xylol.

Vorzugsweise wird die Reaktion bei einer Temperatur im Bereich von -30°C bis 80°C durchgeführt, besonders bevorzugt bei einer Temperatur im Bereich von 10°C bis 50°C, insbesondere im Bereich von 20°C bis 30°C.

In einer bevorzugten Ausführungsform der Erfindung steht R für eine geradkettige oder verzweigte C₁-C₈-Alkyl- oder -Alkenylgruppe, zum Beispiel eine Methyl-, Ethyl-, n-Propyl-, Isopropyl-, n-Butyl-, sec-Butyl-, tert-Butyl-, Pentyl-, Hexyl-, Heptyl-, Octyl-, 2-Ethylhexyl-, Allyl- oder Vinylgruppe. Besonders bevorzugt steht R für eine Ethyl-, n-Butyl- oder 2-Ethylhexylgruppe.

Die Erfindung betrifft weiterhin die Herstellung von 2-Cyclododecyl-1-propanol umfassend die Herstellung eines tertiären Alkohols der Formel (I) nach einem der vorangehenden Ansprüche und die Umsetzung des so hergestellten tertiären Alkohols der Formel (I) zu 2-Cyclodoceyl-1-propanol.

Statt der sowohl im Hinblick auf die Materialkosten als auch die Abwasserproblematik nicht optimalen Reformatsky-Reaktion mit Zink wird erfindungsgemäß eine reduktive Aldol-Reaktion unter Metall-Katalyse, insbesondere Kupfer-Katalyse, für die erste Stufe der Synthese von 2-Cyclododecyl-1-propanol eingesetzt.

Das erfindungsgemäße Verfahren lässt sich schematisch wie folgt darstellen:

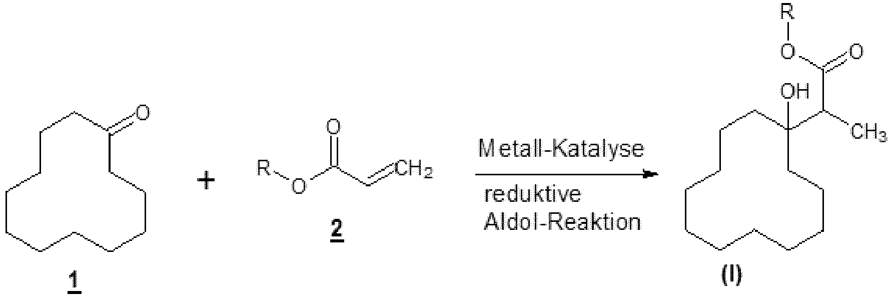

Wie aus dem obigen Reaktionsschema ersichtlich, erhält man aus der reduktiven Aldol-Reaktion von Cyclododecanon **1** mit dem Acrylat **2** den tertiären Alkohol **(I).**

Der erhaltene tertiäre Alkohol mit der Formel **(I)** entspricht dem Produkt aus der Reformatsky-Reaktion, wie sie in der DE 37 03 585 beschrieben ist, und dient dann weiter zur Herstellung von 2-Cyclododecyl-1-propanol.

Das erfindungsgemäße Verfahren liefert den tertiären Alkohol der Formel **(I)** stabil in einem Umsatz von mehr als 75%.

Für die Katalyse der reduktiven Aldol-Reaktion kommen verschiedene Metall-Katalysatoren in Betracht, beispielsweise Katalysatoren auf der Basis von Rhodium, Palladium, Cobalt oder Zink, wie in der Literatur zu diesem Reaktionstypus beschrieben. Als besonders vorteilhaft im Rahmen der vorliegenden Erfindung haben sich allerdings Kupfer-Katalysatoren erwiesen und insbesondere die Verbindung Cu(OAc)₂ × H₂O.

Bei der Kupfer-Katalyse läuft die reduktive Aldol-Reaktion üblicherweise in Gegenwart eines Silans ab. Bei anderen Metall-Katalysatoren, wie beispielsweise Rhodium-Verbindungen, kann die Reaktion aber auch in einer Wasserstoff-Atmosphäre als Reduktionsmittel durchgeführt werden.

Wenn die reduktive Aldol-Reaktion unter Kupfer-Katalyse in Gegenwart eines Silans durchgeführt wird - wie gegenwärtig bevorzugt -, erfolgt die Spaltung der O-Si-Bindung der in einem Zwischenschritt entstehenden Siloxanverbindung üblicherweise mit Säure, um zum tertiären Alkohol mit der Formel (I) zu gelangen. Für diese Spaltungsreaktion kämen aber auch andere Reagenzien in Betracht wie beispielsweise Fluor-Verbindungen, z.B. Ammoniumfluorid oder Tetrabutylammoniumfluorid (TBAF). In einer besonders bevorzugten Ausführungsform der Erfindung wird als Säure HCl, H₂SO₄ oder H₃PO₄ verwendet. Aber auch andere Säuren, wie HNO₃ oder para-Toluolsulfonsäure, können eingesetzt werden. Es kommen auch Basen wie Natronlauge und Kalilauge in Betracht, wenn die basischen Bedingungen mild bleiben, da sich ansonsten der tertiäre Alkohol zersetzen würde.

In einer bevorzugten Ausführungsform der Erfindung können Liganden für den Katalysator eingesetzt werden, wie bspw. bidentate Liganden, wie sie in der Literatur zu reduktiven Aldol-Reaktionen beschrieben sind (Organic Letters, 2005, Vol. 7, No. 19, Seiten 4225-4228; Beilstein Journal of Organic Chemistry, 2015, 11, Seiten 213 bis 218; Tetrahedron Letters 47, 2006, Seiten 1403-1407). Der Ligand kann auch ausgewählt werden aus der Gruppe der Organophosphor-Verbindungen, wie beispielsweise der Phosphine, Diphosphine, Diamine oder der organischen Phosphite. Erfindungsgemäß hat sich der Einsatz von Triphenylphosphin als Ligand als besonders vorteilhaft erwiesen.

Falls ein Lösungsmittelsystem eingesetzt wird, umfasst dieses bevorzugt Toluol oder Xylol und besteht in einer besonderen Ausführungsform vollständig daraus.

Um die unerwünschte Polymerisation von Acrylat zu vermindern, kann in einer bevorzugten Ausführungsform des erfindungsgemäßen Verfahrens ein Stabilisator eingesetzt werden, vorzugsweise Hydrochinonmonomethylether. Die unerwünschte Polymerisation des Acrylats kann zusätzlich aber auch durch die Auswahl des Acrylats minimiert werden.

Der mit dem erfindungsgemäßen Verfahren hergestellte tertiäre Alkohol der Formel **(I)** kann dann in per se bekannten weiteren Reaktionsstufen zum Endprodukt 2-Cyclododecyl-1-propanol umgesetzt werden, wie beispielsweise in der DE 37 03 585 A1 beschrieben.

### Detaillierte Darstellung der Erfindung

Das erfindungsgemäße Verfahren wird anhand des nachfolgenden Beispiels näher erläutert, das ausschließlich der Veranschaulichung dienen und den Schutzumfang des Patentes nicht einschränken soll.

### Beispiel

Ein 2 Liter-Dreihals-Kolben mit Magnetrührer und Tropftrichter wird durch dreimaliges Anlegen von Vakuum und Spülen mit N₂ inertisiert. Im N₂-Gegenstrom werden 1070 ml Toluol zugegeben, gefolgt von 2,04 g Hydrochinonmonomethylether, 4,93 g Cu(OAc)₂ × H₂O und 300 g (pulverisiertem) Cyclododecanon. Anschließend werden 16,19 g Triphenylphosphin zugegeben.

Anschließend werden bei Raumtemperatur langsam mittels Tropftrichter 225,3 g PMHS zugegeben und die Mischung dann bei Raumtemperatur für 15 min rühren gelassen.

Dann wird der Ansatz auf 30°C erwärmt und mittels eines Tropftrichters werden langsam 173 g Ethylacrylat zugetropft. Nach Zugabe von circa 5 g Ethylacrylat springt die Reaktion an, erkennbar an einem Anstieg der Innentemperatur. Beim weiteren Zutropfen des Ethylacrylats über einen Zeitraum von 3,5 h wird die Innentemperatur des Ansatzes (gegebenenfalls unter Kontrolle mit einem Wasserbad) zwischen 25°C und 35°C gehalten. Nach kompletter Zugabe des Ethylacrylats wird der Ansatz für 18 h bei Raumtemperatur nachgerührt.

Zur intermediär gebildeten Polymethylsiloxan-Verbindung werden ohne Aufarbeitung über einen Zeitraum von 2 h 500 ml konz. HCl (32 %ig) zugetropft, um den entsprechenden tertiären Alkohol zu erhalten. Die Innentemperatur steigt dabei anfangs von 22°C auf 25°C an. Nach vollendeter Zugabe der Salzsäure wird der Reaktionsansatz für 18 h bei Raumtemperatur gerührt. Der Ansatz wird in einen Scheidetrichter überführt und die Phasen getrennt.

Vom tertiären Alkohol wird zur Charakterisierung eine kleine Probe genommen und mittels GC analysiert. Dabei zeigte sich, das der gebildete tertiäre Alkohol identisch war mit dem tertiären Alkohol, der aus einer entsprechenden Reformatsky-Reaktion aus Cyclododecanon und 2-Brompropionsäureethylester in Gegenwart von Zink gemäß DE 3703585 A1 (Beispiel 1, jedoch ohne die Eliminerung von Wasser aus dem tertiären Alkohol ) erhalten wird.

Charakterisierungsdaten: Im GC-FID konnten neben dem gewünschten tertiären Alkohol noch nicht-umgesetztes Cyclododecanon, Cyclododecanol und Triphenylphosphin sowie weitere, unbekannte Substanzen nachgewiesen werden.

## Patentansprüche

1. Verfahren zur Herstellung eines tertiären Alkohols mit der Formel (I) 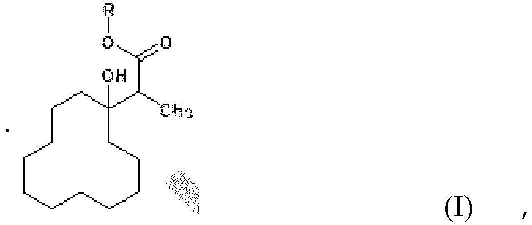 bei dem Cyclododecanon mit einem Acrylat mit der Formel RO-CO-CH=CH₂ in einer reduktiven Aldol-Reaktion unter Metall-Katalyse umgesetzt wird,
wobei R in der obigen Formel (I) für eine geradkettige oder verzweigte C₁-C₈-Alkyl- oder -Alkenylgruppe oder eine unsubstituierte oder substituierte Phenylgruppe steht.

2. Verfahren nach Anspruch 1, **dadurch gekennzeichnet, dass** die Reaktion unter Kupfer-Katalyse in Gegenwart eines Silans durchgeführt wird.

3. Verfahren nach Anspruch 2, **dadurch gekennzeichnet, dass** als Silan Polymethylhydrosiloxan (PMHS) verwendet wird.

4. Verfahren nach Anspruch 2 oder 3, **dadurch gekennzeichnet, dass** als Katalysator Cu(OAc)₂ × H₂O verwendet wird.

5. Verfahren nach einem der vorangehenden Ansprüche, **dadurch gekennzeichnet, dass** eine Phosphinverbindung als Ligand des Katalysators verwendet wird.

6. Verfahren nach Anspruch 5, **dadurch gekennzeichnet, dass** als Ligand Triphenylphosphin verwendet wird.

7. Verfahren nach einem der vorangehenden Ansprüche, **dadurch gekennzeichnet, dass** für das Acrylat ein Stabilisator verwendet wird.

8. Verfahren nach Anspruch 7, **dadurch gekennzeichnet, dass** als Stabilisator für das Acrylat Hydrochinonmonomethylether verwendet wird.

9. Verfahren nach einem der vorangehenden Ansprüche, **dadurch gekennzeichnet, dass** die Reaktion in einem Lösungsmittelsystem durchgeführt wird.

10. Verfahren nach Anspruch 9, **dadurch gekennzeichnet, dass** das Lösungsmittelsystem Toluol oder Xylol umfasst.

11. Verfahren nach einem der vorangehenden Ansprüche, **dadurch gekennzeichnet, dass** die Reaktion bei einer Temperatur im Bereich von -30°C bis 80°C, bevorzugt im Bereich von 10°C bis 50°C, besonders bevorzugt im Bereich von 20°C bis 30°C durchgeführt wird.

12. Verfahren nach einem der vorangehenden Ansprüche, **dadurch gekennzeichnet, dass** R für eine geradkettige oder verzweigte C₁-C₈-Alkyl- oder -Alkenylgruppe steht.

13. Verfahren nach Anspruch 12, **dadurch gekennzeichnet, dass** R für eine Ethyl-, n-Butyl- oder 2-Ethylhexylgruppe steht.

14. Herstellung von 2-Cyclododecyl-1-propanol umfassend die Herstellung eines tertiären Alkohols der Formel (I) nach einem der vorangehenden Ansprüche und die Umsetzung des so hergestellten tertiären Alkohols der Formel (I) zu 2-Cyclodoceyl-1-propanol.

## Claims

1. Method for preparing a tertiary alcohol having the formula (I) 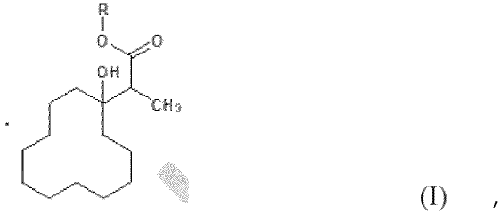
in which cyclododecanone is reacted with an acrylate having the formula RO-CO-CH=CH₂ in a reductive aldol reaction with metal catalysis,
wherein R in the above formula (I) stands for a straight-chain or branched C₁-C₈ alkyl group or alkenyl group or an unsubstituted or substituted phenyl group.

2. Method according to claim 1, **characterised in that** the reaction is carried out under copper catalysis in the presence of a silane.

3. Method according to claim 2, **characterised in that** polymethylhydrosiloxane (PMHS) is used as silane.

4. Method according to claim 2 or 3, **characterised in that** Cu(OAc)₂ × H₂O is used as catalyst.

5. Method according to any one of the preceding claims, **characterised in that** a phosphine compound is used as ligand of the catalyst.

6. Method according to claim 5, **characterised in that** triphenylphosphine is used as ligand.

7. Method according to any one of the preceding claims, **characterised in that** a stabiliser is used for the acrylate.

8. Method according to claim 7, **characterised in that** hydroquinone monomethyl ether is used as stabiliser for the acrylate.

9. Method according to any one of the preceding claims, **characterised in that** the reaction is carried out in a solvent system.

10. Method according to claim 9, **characterised in that** the solvent system comprises toluene or xylene.

11. Method according to any one of the preceding claims, **characterised in that** the reaction is carried out at a temperature in the range of from -30°C to 80°C, preferably in the range of from 10°C to 50°C, particularly preferably in the range of from 20°C to 30°C.

12. Method according to any one of the preceding claims, **characterised in that** R stands for a straight-chain or branched C₁-C₈ alkyl group or alkenyl group.

13. Method according to claim 12, **characterised in that** R stands for an ethyl group, n-butyl group or 2-ethylhexyl group.

14. Preparation of 2-cyclododecyl-1-propanol comprising the preparation of a tertiary alcohol of formula (I) according to any one of the preceding claims and the reaction of the thus prepared tertiary alcohol of formula (I) to form 2-cyclododecyl-1-propanol.

## Revendications

1. Procédé de préparation d'un alcool tertiaire avec la formule (I) 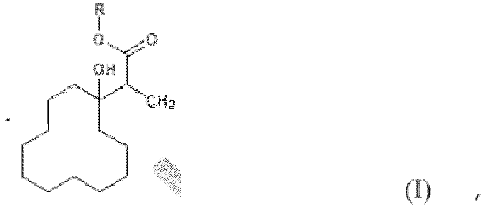
où du cyclododécanone est mis en réaction avec un acrylate de la formule RO-CO-CH=CH₂ dans une réaction aldolique réductive par catalyse métallique,
dans lequel R dans la formule ci-dessus (I) est un groupe alkyle ou alcényle en C₁-C₈ à chaîne droite ou ramifié ou un groupe phényle non substitué ou substitué.

2. Procédé selon la revendication 1, **caractérisé en ce que** la réaction est mise en oeuvre en présence d'un silane par catalyse par le cuivre.

3. Procédé selon la revendication 2, **caractérisé en ce qu'**est utilisé en tant que silane du polyméthylhydrosiloxane (PMHS).

4. Procédé selon la revendication 2 ou 3, **caractérisé en ce qu'**est utilisé en tant que catalyseur du Cu(OAc)₂ × H₂O.

5. Procédé selon l'une quelconque des revendications précédentes, **caractérisé en ce qu'**un composé de phosphine est utilisé en tant que ligand du catalyseur.

6. Procédé selon la revendication 5, **caractérisé en ce qu'**est utilisée en tant que ligand de la triphénylphosphine.

7. Procédé selon l'une quelconque des revendications précédentes, **caractérisé en ce qu'**un agent stabilisant est utilisé pour l'acrylate.

8. Procédé selon la revendication 7, **caractérisé en ce que** du monométhyléther d'hydroquinone est utilisé en tant qu'agent stabilisant pour l'acrylate.

9. Procédé selon l'une quelconque des revendications précédentes, **caractérisé en ce que** la réaction est mise en oeuvre dans un système solvant.

10. Procédé selon la revendication 9, **caractérisé en ce que** le système solvant comprend du toluène ou du xylène.

11. Procédé selon l'une quelconque des revendications précédentes, **caractérisé en ce que** la réaction est mise en oeuvre à une température dans la plage de -30 °C à 80 °C, de manière préférée dans la plage de 10 °C à 50 °C, de manière particulièrement préférée dans la plage de 20 °C à 30 °C.

12. Procédé selon l'une quelconque des revendications précédentes, **caractérisé en ce que** R est un groupe alkyle ou alcényle en C₁-C₈ à chaîne droite ou ramifié.

13. Procédé selon la revendication 12, **caractérisé en ce que** R est un groupe éthyle, n-butyle ou 2-éthylhexyle.

14. Préparation de 2-cyclododécyl-1-propanol comprenant la préparation d'un alcool tertiaire de la formule (I) selon l'une quelconque des revendications précédentes et la mise en réaction de l'alcool tertiaire ainsi produit de la formule (I) en 2-cyclododécyl-1-propanol.
